# EUROPEAN PATENT SPECIFICATION

(11) **EP 0 735 862 B1**
(45) Date of publication and mention of the grant of the patent: **17.07.2002**
(21) Application number: 95906013.8
(22) Date of filing: 20.12.1994
(51) Int. Cl.: A61K 9/20, A61K 9/16

(54) **METHOD FOR DRY BLEND COMPRESSION OF MEDICAMENTS**
VERFAHREN ZUR TROCKENMISCHUNGKOMPRESSION VON ARZNEIMITTELN
PROCEDE DE COMPRESSION D'UN MELANGE SEC DE MEDICAMENTS

(30) Priority: 21.12.1993 ZA 9309566
(43) Date of publication of application: 09.10.1996
(73) Proprietor: APPLIED ANALYTICAL INDUSTRIES, INC., Wilmington, NC 28405 (US)
(72) Inventor: GREAVES, Frank C., Wilmington, NC 28409 (US); SWARBRICK, James, Hampstead, NC 28443 (US); BEASLEY, Martin W., Wilmington, NC 28409 (US)
(74) Representative: Ahner, Francis
(86) International application number: US9414639
(87) International publication number: WO9517169

(56) References cited:
- EP-A- 0 179 703
- EP-A- 0 503 521

## Description

### Technical Field

This invention relates to a method of dry blend compression of insoluble potent drug substances using a directly compressible, agglomerated excipient that is not a conventional spray dried polyalcohol or lactose.

### Background Art

Inadequate distribution of low-dose potent drugs is a constant threat to the uniform potency of tablets and capsules containing such drugs.

The greatest potential for drug-diluent segregation in a tablet system occurs with powder or particulate mixtures intended for direct compression or wet granulation in which the drug migration occurs (Dale E. Fonner et. al., Pharmaceutical Dosage Forms: Tablets, Volume 2, pp. 253.). European Patent Application 92103963.2, AKZO N.V. p. 6 describes the inadequate homogeneity encountered with compositions containing hydrous lactose DT (U.S. Patent 4,544,554 etc. issued to Samuel A. Pasquale). The migration of a low-dose potent drug disrupts the distribution throughout the mix, giving rise to inconsistency in the content uniformity of the dosage form.

U.S. Patent No. 3,568,828 issued to Leonard Joseph Lerner describes wet processes using potent drug substances such as estrogens with organic solvents such as chloroform. Such processes are now regarded as environmentally unsafe and can incur considerable manufacturing expenses, in that appropriate solvent scrubbing and/or explosion proof equipment must be acquired at substantial capital expenditure.

EP-A-0 179 703 describes a method by which mannitol can be processed to make it directly compressible and to give it a friability suitable for shipping. This is done by giving the mannitol a particular granulometry.

Estradiol and a number of other low-dose potent drugs precipitate in a variety of polymorphs and/or crystal habits. The changes in the crystal structure on drying can affect the bioavailability of the drug. It is well known in the literature that the micronized form is more bioavailable than larger drug particle size. This invention offers an important alternative to wet granulation, thus eliminating recrystallization and the issue of polymorphism and bioavailability. It also offers the choice of dry mixing or direct compression with materials other than the conventional spray dried polyalcohols or lactoses.

### Disclosure the Invention

The following describes the use of low dose medicinal agents such as micronized steroidal medicinal agents; estradiol, equilin, estrone, spironolactone, and non-micronized materials; such as, estropipate, conjugated estrogens, esterified estrogens, progestins or other medicinal agents having the structure which includes the cyclopentanoperhydrophenanthrene ring system which agents are formulated by a drug pharmaceutical preparation. The dry preparation makes use of excipients that have been prepared by agglomeration methods other than by spray drying. Such excipients include granular mannitol, agglomerated maltodextrin, corn syrup solids and mixtures of these agents with added conventional direct compression excipients.

The active ingredients comprise any medicament which has a low effective dose such as those below 10 mg per dosage unit. Most useful are those medicaments having a steroidal nucleus, the cyclopentanoperhydrophenanthrene ring system, in their chemical structures such as the estrogens or progestins.

Examples of the former are ethinylestradiol, estrone, mestranol, 17-alpha-ethinyl estradiol-3-methylether, esterified estrogens, and, especially estradiol, conjugated estrogens, methyl testosterone. The dosage amounts and indications of these and other active ingredients are those described in the literature such as the Physician's Desk Reference.

The progestins are 3-ketodesogestrel, desogestrel, levo-desogestrel, norgestrel, gestodene, norethindrone, norethindrone acetate.

The agglomerated excipients used in this method are those listed above. These are differentiated from those used in the AKZO method of reference in not having the AKZO prescribed affinity-demixing range of properties of active ingredient to dry excipient. The latter property gives the method of this invention a commercial advantage in manufacturing procedure.

Studies comparing the properties of the AKZO preferred excipient (spray dried lactose) with applicant's lead species (agglomerated mannitol) demonstrated that the former retained 2 to 3 times the quantity of estradiol on its surface than did the claimed ingredient. The tablet of this invention, however, gave good drug distribution figures despite not having the high affinity AKZO states to be necessary.

Also, photomicrographs of the samples demonstrated that the AKZO mix had drug uniformly attached to all the surfaces of the excipient. In contrast, the mix of this invention had drug located in crevices of the agglomerate. This indicates a different mechanism of attachment.

The method of this invention, therefore, comprises mixing one or more low-dose medicaments with the agglomerated excipient, usually in a ratio of from 1:40 to 1:100, medicament to excipient. Other excipients, disintegrating agents or lubricants are optionally added. After dry mixing, the mix is compressed into tablets.

The following examples illustrate the method of this invention in more detail.

### Example I

| ESTRADlOL 2 mg TABLETS | | | |
|---|---|---|---|
| Materials | A (%) (comparative) | B (%) | C (%) |
| Microcrystalline Cellulose (Avicel PHI 02) | 45.89 | 23.20 | |
| Starch 1500 | - | 20.00 | 20.00 |
| Dibasic Calcium Phosphate (Cal-Star) | - | - | 23.20 |
| Agglomerated Mannitol | 47.39 | 50.08 | 50.08 |
| Croscarmellose Sodium (Ac-Di-Sol) | 5.00 | 5.00 | 5.00 |
| Magnesium Stearate | 0.50 | 0.50 | 0.50 |
| Estradiol | 1.22 | 1.22 | 1.22 |
| 1. Screen estradiol and mannitol together through a #20 mesh screen into a slant-cone high speed mixer. | | | |
| 2. Screen Avicel PH 102 and Ac-Di-Sol through the screen and force any remaining estradiol through as well. | | | |
| 3. Blend for fourteen (14) minutes with the agitator bar off, two (2) minutes with the agitator bar on, and blend fourteen (14) minutes with the agitator bar off (total of thirty (30) minutes). | | | |
| 4. Add the magnesium stearate and blend for three (3) minutes with the agitator bar off. | | | |
| 5. Compress into tablets on a high speed tablet press. Tablet weight of 164 mg± 3%. | | | |

Other illustrations are:
1. Using the process of example 1 with agglomerated maltodextrin blended with estradiol and a progestin.
2. Using the process of example 1 with a combination of agglomerated mannitol and maltodextrin blended with an estrogen or a progestin or a combination of estrogen and progestin.
3. Using the process of example 1 with a combination of agglomerated mannitol and maltodextrin blended with conjugated estrogens alone or in combination with a progestin.

## Claims

1. Method of making pharmaceutical tablets, **characterised in that** it comprises the steps consisting of blending a unit dosage quantity of one or more low dosage medicaments, wherein the medicament is chosen from the group comprising estrogens and progestins, with an agglomerated excipient in a ratio of from 1:40 to 1:100 of medicament to excipient, wherein the excipient is selected from the group comprising mannitol, maltodextrin, corn syrup solid, or mixtures thereof, and optionally, one or more tableting aids, and compressing the blended mixture.

2. Method of claim 1, **characterised in that** said estrogen is estradiol.

3. Method of claim 1, **characterised in that** said estrogen is a conjugated estrogen.

4. Method of claim 1, **characterised in that** said progestin is selected from the group consisting of 3-ketodesogestrel, desogestrel, levodesogestrel, norgestrel, gestodene, norethindrone, norethindrone acetate.

5. Method of any of claims 1 to 4, **characterised in that** the excipient is mannitol, maltodextrin, or a combination thereof.

6. Pharmaceutical composition when made by a method as claimed in anyone of claims 1 to 4.

7. Pharmaceutical composition of claim 6, **characterised in that** the excipient is mannitol, maltodextrin or a combination thereof.

8. Pharmaceutical composition, **characterised in that** it comprises one or more low dosage medicaments, wherein the medicament is chosen from the group comprising estrogens and progestins and an agglomerated excipient in a ratio of from 1:40 to 1:100 of medicament to excipient, wherein the excipient is selected from the group consisting of mannitol, maltodextrin, corn syrup solid and mixtures thereof, and optionally, one or more tableting aids.

9. Composition of claim 8, **characterised in that** said estrogen is estradiol.

10. Composition of claim 8, **characterised in that** said estrogen is a conjugated estrogen.

11. Composition of claim 8, **characterised in that** said progestin is selected from the group consisting of 3-ketodesogestrel, desogestrel, levodesogestrel, norgestrel, gestodene, norethindrone, norethindrone acetate.

12. Composition of anyone of claims 8 to 11, **characterised in that** the excipient is mannitol, maltodextrin or a combination thereof.

13. Composition of anyone of claims 8 to 11, **characterised in that** said composition is in the form of a tablet.

14. Composition of claim 13, **characterized in that** said progestin is selected from the group consisting of 3-ketodesogestrel, desogestrel, levodesogestrel, norgestrel, gestodene, norethindrone, norethindrone acetate, and mixtures thereof.

15. Composition of any one of claims 9 to 14, **characterized in that** the excipient is mannitol, maltodextrin, or a combination thereof.

16. Composition of any one of claims 9 to 14, **characterized in that** said composition is in the form of a tablet.

## Patentansprüche

1. Verfahren zur Herstellung pharmazeutischer Tabletten, **dadurch gekennzeichnet, daß** es die Schritte umfaßt, welche aus dem Mischen einer Dosierungseinheitsmenge von einem oder mehreren niedrig dosierten Medikament(en), wobei das Medikament aus der Gruppe umfassend Estrogene und Progestine ausgewählt ist, mit einem agglomerierten Träger in einem Verhältnis von 1:40 bis 1:100 von Medikament zu Träger, wobei der Träger aus der Gruppe umfassend Mannit, Maltodextrin, verfestigten Maisstärkezuckersirup oder Mischungen davon ausgewählt ist, und gegebenenfalls einem oder mehreren Tablettierhllfsstoff(en) und Pressen der gemischten Mischung bestehen.

2. Verfahren nach Anspruch 1, **dadurch gekennzeichnet, daß** das Estrogen Estradiol ist.

3. Verfahren nach Anspruch 1, **dadurch gekennzeichnet, daß** das Estrogen ein konjugiertes Estrogen ist.

4. Verfahren nach Anspruch 1, **dadurch gekennzeichnet, daß** das Progestin aus der Gruppe bestehend aus 3-Ketodesogestrel, Desogestrel, Levodesogestrel, Norgestrel, Gestoden, Norethindron, Norethindronacetat ausgewählt ist.

5. Verfahren nach irgendeinem der Ansprüche 1 bis 4, **dadurch gekennzeichnet, daß** der Träger Mannit, Maltodextrin oder eine Kombination davon ist.

6. Pharmazeutische Zusammensetzung, die nach einem Verfahren wie in irgendeinem der Ansprüche 1 bis 4 beansprucht hergestellt ist.

7. Pharmazeutische Zusammensetzung nach Anspruch 6, **dadurch gekennzeichnet, daß** der Träger Mannit, Maltodextrin oder eine Kombination davon ist.

8. Pharmazeutische Zusammensetzung, **dadurch gekennzeichnet, daß** sie ein oder mehrere niedrigdosierte(s) Medikament(e), wobei das Medikament aus der Gruppe umfassend Estrogene und Progestine ausgewählt ist, und einen agglomerierten Träger in einem Verhältnis von 1:40 bis 1:100 von Medikament zu Träger, wobei der Träger aus der Gruppe bestehend aus Mannit, Maltodextrin, verfestigtem Maisstärkezuckersirup und Mischungen davon ausgewählt ist, und gegebenenfalls einen oder mehrere Tablettierhilfsstoff(e) enthält.

9. Zusammensetzung nach Anspruch 8, **dadurch gekennzeichnet, daß** das Estrogen Estradiol ist.

10. Zusammensetzung nach Anspruch 8, **dadurch gekennzeichnet, daß** das Estrogen ein konjugiertes Estrogen ist.

11. Zusammensetzung nach Anspruch 8, **dadurch gekennzeichnet, daß** das Progestin aus der Gruppe bestehend aus 3-Ketodesogestrel, Desogestrel, Levodesogestrel, Norgestrel, Gestoden, Norethindron, Norethindronacetat ausgewählt ist.

12. Zusammensetzung nach irgendeinem der Ansprüche 8 bis 11, **dadurch gekennzeichnet, daß** der Träger Mannit, Maltodextrin oder eine Kombination davon ist.

13. Zusammensetzung nach irgendeinem der Ansprüche 8 bis 11, **dadurch gekennzeichnet, daß** die Zusammensetzung in Form einer Tablette vorliegt.

14. Zusammensetzung nach Anspruch 13, **dadurch gekennzeichnet, daß** das Progestin aus der Gruppe bestehend aus 3-Ketodesogestrel, Desogestrel, Levodesogestrel, Norgestrel, Gestoden, Norethindron, Norethindronacetat und Mischungen davon ausgewählt ist.

15. Zusammensetzung nach irgendeinem der Ansprüche 9 bis 14, **dadurch gekennzeichnet, daß** der Träger Mannit, Maltodextrin oder eine Kombination davon ist.

16. Zusammensetzung nach irgendeinem der Ansprüche 9 bis 14, **dadurch gekennzeichnet, daß** die Zusammensetzung in Form einer Tablette vorliegt.

## Revendications

1. Procédé de fabrication de comprimés pharmaceutiques, **caractérisé en ce qu'**elle comprend les étapes consistant à mélanger une quantité unitaire de posologie dans plusieurs médicaments à faible dosage, dans lequel le médicament est choisi parmi le groupe comprenant les oestrogènes et les progestatifs, avec un excipient aggloméré dans un rapport de 1 : 40 à 1 : 100 de médicament par rapport à l'excipient, dans lequel l'excipient est choisi parmi le groupe comprenant le mannitol, la maltodextrine, le sirop de maïs solide ou des mélanges de ceux-ci, et de façon optionnelle, un ou plusieurs auxiliaires de mise en comprimé, et en compressant le mélange malaxé.

2. Procédé selon la revendication 1, **caractérisé en ce que** ledit oestrogène est l'oestradiol.

3. Procédé selon la revendication 1, **caractérisé en ce que** ledit oestrogène est un oestrogène conjugué.

4. Procédé selon la revendication 1, **caractérisé en ce que** ledit progestatif est choisi parmi le groupe constitué du 3-cétodésogestrel, du désogestrel, du lévonorgestrel, du norgestrel, du gestodène, du noréthistérone, de l'acétate de noréthistérone.

5. Procédé selon l'une quelconque des revendications 1 à 4, **caractérisé en ce que** l'excipient est le mannitol, la maltodextrine ou une combinaison de ceux-ci.

6. Composition pharmaceutique quand elle est faite par un Procédé tel que revendiqué selon l'une quelconque des revendications 1 à 4.

7. Composition pharmaceutique selon la revendication 6, **caractérisée en ce que** l'excipient est le mannitol, la maltodextrine ou une combinaison de ceux-ci.

8. Composition pharmaceutique, **caractérisée par** ce qu'elle comprend un ou plusieurs médicaments à faible dosage, dans laquelle le médicament est choisi parmi le groupe constitué des oestrogènes et des progestatifs, et un excipient aggloméré dans un rapport de 1 : 40 à 1 : 100 de médicament par rapport l'excipient, dans laquelle l'excipient est choisi parmi le groupe constitué du mannitol, de la maltodextrine, du sérum de maïs solide et des mélanges de ceux-ci, et de façon optionnelle, un ou plusieurs auxiliaires de mise en comprimés.

9. Composition selon la revendication 8, **caractérisée en ce que** ledit oestrogène est l'oestradiol.

10. Composition selon la revendication 8, **caractérisée en ce que** ledit oestrogène est un oestrogène conjugué.

11. Composition selon la revendication 8, **caractérisée en ce que** ledit progestatif est choisi parmi le groupe constitué du 3-cétodésogestrel, du désogestrel, du lévonorgestrel, du norgestrel, du gestodène, du noréthistérone, de l'acétate de noréthistérone.

12. Composition selon l'une quelconque des revendications 8 à 11, **caractérisée en ce que** l'excipient est le mannitol, la maltodextrine ou une combinaison de ceux-ci.

13. Composition selon l'une quelconque des revendications 8 à 11, **caractérisée en ce que** ladite composition est sous la forme d'un comprimé.

14. Composition selon la revendication 13, **caractérisée en ce que** ledit progestatif est choisi parmi le groupe constitué du 3-cétodésogestrel, du désogestrel, du lévonorgestrel, du norgestrel, le gestodène, du noréthistérone, de l'acétate de noréthistérone et des mélanges de ceux-ci.

15. Composition selon l'une quelconque des revendications 9 à 14, **caractérisée en ce que** l'excipient est le mannitol, la maltodextrine ou une combinaison de ceux-ci.

16. Composition selon l'une quelconque des revendications 9 à 14, **caractérisée en ce que** ladite composition est sous la forme d'un comprimé.
